# EUROPÄISCHE PATENTANMELDUNG

(11) **EP 0 672 757 A1**
(43) Veröffentlichungstag der Anmeldung: **20.09.1995**
(21) Anmeldenummer: 95103463.6
(22) Anmeldetag: 10.03.1995
(51) Int. Cl.: C12Q 1/04, C12Q 1/68, C07K 14/705

(54) **Funktionelles Testverfahren zur Identifizierung von Thrombinrezeptor-Antagonisten**

(30) Priorität: 16.03.1994 DE 4408891
(71) Anmelder: BASF AKTIENGESELLSCHAFT, D-67056 Ludwigshafen (DE)
(72) Erfinder: Kröger, Burkhard, Dr., D-67117 Limburgerhof (DE); Bialojan, Siegfried, Dr., D-68723 Oftersheim (DE); Endler-Jobst, Anni Barbara, Dr., D-69469 Weinheim (DE)

(57) **Zusammenfassung**

Die Erfindung betrifft ein Testverfahren zur Identifizierung von Substanzen, die die Aktivierung des Thrombinrezeptors blockieren, welches folgende Schritte umfaßt:
a) Wahl einer Zellinie, die einen Thrombinrezeptor endogen exprimiert oder Herstellen einer Zellinie, die einen Thrombinrezeptor rekombinant stabil exprimiert,
b) Transfektion einer Wirtszelle nach Schritt a) mit einem rekombinanten Vektor, der
   b1) einen Promotor enthält, der durch Erhöhung intrazellulärer Botenstoffe aktiviert wird, und
   b2) ein Reportergen, funktionell mit dem in b1) beschriebenen Promotor verknüpft, trägt.
c) Etablieren von Zellen nach Schritt b), die
   c1) den unter Schritt b) beschriebenen Vektor transient enthalten, oder
   c2) den unter Schritt b) beschriebenem Vektor stabil integriert enthalten,
d) Exposition der Zellen aus Schritt c) mit der zu testenden Substanz,
e) Messung der Expression des Reportergens in den Zellen nach Schritt d).

## Beschreibung

Die vorliegende Erfindung betrifft ein Testverfahren zur Identifizierung von Thrombinrezeptor-Antagonisten.

Thrombin ist das zentrale Enzym der Blutgerinnungskaskade. Es bewirkt eine Reihe von Aktivitäten, die sowohl unter natürlichen Umständen,z.B. nach Verletzungen von Blutgefäßen bedeutsam sind, als auch bei pathologischen Veränderungen des Gefäßsystems eine große Rolle spielen.

Die Protease Thrombin katalysiert nicht nur die Umwandlung löslichen Fibrinogens in das unlösliche Fibrin, sie ist gleichzeitig auch ein Aktivator der Blutplättchen-Aggregation. Tierversuche zeigen, daß eben diese Aktivität die Ursache pathologischer arterieller Thrombusbildung sein kann. Die Natur der Wechselwirkung zwischen der Protease Thrombin und dem Thrombinrezeptor auf den Blutplättchen war lange Gegenstand von Spekulationen. Ob es sich hier um einen reinen Bindungsmechanismus oder die proteolytische Veränderung des Rezeptors handelt, wurde durch die Klonierung des humanen Thrombinrezeptors durch Vu et al. (Cell 64, 1057-1068, 1991) aufgeklärt.

Der Thrombinrezeptor ist ein Mitglied der sogenannten 7-Transmembran-Domänen-Rezeptor Familie. Sein Aminoterminus trägt extrazellulär eine Consensus-Spaltstelle für Thrombin. Die proteolytische Spaltung durch Thrombin an dieser Stelle demaskiert einen neuen Aminoterminus, der nun als sogenannter "tethered ligand" den Rezeptor aktiviert. Eine Substanz, die die Rezeptoraktivierung verhindern könnte, wäre von großem therapeutischem Nutzen. Denkbar wäre der Einsatz bekannter Thrombininhibitoren, wie Hirudin oder D-Phe-Pro-Arg-chloromethylketon. Bei diesen handelt es sich aber um "active-site" Inhibitoren des Thrombins, die jegliche, auf Proteolyse basierende Aktivität des Thrombins verhindern, so zum Beispiel auch die Spaltung des Fibrinogens oder die Aktivierung des Proteins C.

Daher ist es wünschenswert, Thrombinrezeptor-spezifische Antagonisten aufzufinden.

Es bestand daher die Aufgabe, ein Testverfahren aufzufinden, das auf der Grundlage der Blockierung der funktionellen Aktivierung des Rezeptors basiert.

Gegenstand der Erfindung ist ein Testverfahren zur Identifizierung von Substanzen, die die Aktivierung des Thrombinrezeptors blockieren und das die folgenden Schritte umfaßt:
a) Wahl einer Zellinie, die einen Thrombinrezeptor endogen exprimiert oder Herstellen einer Zellinie, die einen Thrombinrezeptor rekombinant stabil exprimiert,
b) Transfektion einer Wirtszelle nach Schritt a) mit einem rekombinanten Vektor, der
   b1) einen Promotor enthält, der durch Erhöhung intrazellulärer Botenstoffe aktiviert wird, und
   b2) ein Reportergen, funktionell mit dem in b1) beschriebenen Promotor verknüpft, trägt.
c) Etablieren von Zellen nach Schritt b), die
   c1) den unter Schritt b) beschriebenen Vektor transient enthalten, oder
   c2) den unter Schritt b) beschriebenem Vektor stabil integriert enthalten,
d) Exposition der Zellen aus Schritt c) mit der zu testenden Substanz,
e) Messung der Expression des Reportergens in den Zellen nach Schritt d).

Mit diesem Verfahren wird der Nachweis von Rezeptor-Antagonisten ermöglicht, deren Grundlage nicht die Thrombininhibition ist.

Die Klasse der 7-Transmembran-Domänen-Rezeptoren ist für gewöhnlich funktionell an G-Proteine gekoppelt. In vielen Fällen ist der von Rezeptor und G-Protein genutzte Effektor die sogenannte Adenylat-Cyclase, deren Produkt der intrazelluläre Botenstoff ("second messenger") cAMP ist. Die Grundlage des Testverfahrens ist die Erfassung der Änderung des cAMP-Spiegels in einer Testzelle mittels eines cAMP-abhängigen Genpromotors und eines gekoppelteten Reportergens. Die Testzelle verfügt dazu über einen Thrombinrezeptor.

Im ersten Schritt des erfindungsgemäßen Testverfahrens wird eine Wirtszelle gewählt, die in ihrer Zytoplasmamembran einen funktionellen Thrombinrezeptor aufweist. Bevorzugt sind dafür Säugerzellen. Diese Zelle kann z.B. durch Transfektion mit einem den Thrombinrezeptor exprimierenden Vektor stabil etabliert werden.

Bevorzugt werden Zellen benutzt, die natürlicherweise einen solchen Rezeptor endogen aufweisen, wie z.B. CHO-Zellen.

In einem zweiten Schritt wird ein rekombinanter Vektor hergestellt, der einen Promotor trägt, welcher abhängig von der Konzentration an intrazellulären Botenstoffen reguliert wird. Unter intrazellulären Botenstoffen oder "second messengers" sind insbesondere Calcium, cAMP, cGMP oder Phosphoinositol-phosphat zu verstehen.

Unter diesem Promoter ist der Sequenzbereich zu verstehen, der alle für die "second messenger"-kontrollierte Transkription notwendigen Elemente umfaßt. Darunter fallen sowohl Elemente der basalen Transkriptionskontrolle, wie die "TATA-box" und die "CCAAT-box", als auch Elemente der induzierbaren Transkriptionskontrolle, wie Bindungssequenzen für "second messenger"-abhängige Transkriptionsfaktoren, sogenannte "response elements" (REs). Hierbei kann es sich um Calcium-REs, cAMP-REs, cGMP-REs oder Phosphoinositolphosphat-REs handeln. Die Sequenz dieser REs und die an sie bindenden Faktoren sind bekannt, so z.B. für das cAMP response element binding protein (CREB) (Montminy, M.R. et al., Proc.Natl.Acad.Sci.USA 83, 6682-6686, 1986).

Die für die Erfindung geeigneten Promotoren können entweder natürlich vorkommende sein, wie z.B. der Promotor des Somatostatingens, des c-fos Gens, des HIV-1 LTRs oder des vasoactive intestinal peptide (VIP) Gens, oder aus mehreren verschiedenen Genpromotoren künstlich zusammengesetzt sein.

Dies kann auch über Fusion und Ligation synthetischer Oligonukleotide erfolgen. Bevorzugt wird die Fusion mehrerer cAMP response elements mit dem basalen Cytomegalovirus IE-Genpromotor durchgeführt.

An diesen zusammengesetzten Promotor wird ein Reportergen funktionell angeknüpft. Reportergene sind solche Gene, deren exprimiertes Genprodukt ein leicht meß- bzw. quantifizierbares Signal liefert.

In der Regel sind geeignete Reportergene Gene für solche Proteine, die mit gängigen Methoden nachweisbar sind. Bevorzugt werden als Reportergene Gene für Enzyme, die eine leicht nachweisbare Reaktion katalysieren, verwendet.

Besonders geeignete Reportergene sind die Gene für Chloramphenicol-Acetyl-Transferase (CAT) oder Luziferase. Die funktionelle Verknüpfung von zusammengesetztem Promotor und Reportergen bedeutet, daß die Transkription des Reportergens unter Kontrolle des "second messenger"-abhängigen Promotors erfolgt.

Neben diesen beiden Genbereichen kann der rekombinante Vektor noch weitere Genabschnitte tragen, so zum Beispiel Regulationssignale oder Selektionsmarker. Besonders vorteilhaft ist die Verwendung eines Selektionsmarkers wie Neomycin-Resistenz (neoR).

Die Transformation einer Wirtszelle durch den rekombinanten Vektor gemäß Schritt b) kann durch alle gängigen Transformationsverfahren, z.B. Elektroporation, Calciumphosphat oder Liposomenvermittelte Transfektion erfolgen. Besonders gut geeignet für die Transformation ist die Liposomenvermittelte Transfektion.

Als Wirtszellen sind eukaryontische Zellinien gut geeignet. Besonders geeignet sind Säugerzellen wie CHO (ATCC Nr. CCL 61).

Nachdem die Wirtszelle mit dem rekombinanten Vektor transformiert worden ist, werden in einem weiteren Schritt c) solche Zellen etabliert, die den Vektor entweder transient oder stabil integriert enthalten.

Unter transienter Expression versteht man eine Expression von nur begrenzter Dauer. Unter stabiler Integration versteht man die Aufnahme des rekombinanten Vektors in das Genom der Wirtszelle.

In einem weiteren Schritt werden die in Schritt c2) etablierten stabilen Zellen den auf ihre antagonisierende Wirkung zu testenden Substanzen ausgesetzt. Gleichzeitig mit der jeweiligen Testsubstanz wird Thrombin, bzw. ein anderer Agonist des Thrombinrezeptors (Thrombinrezeptorpeptide) zugesetzt. Die zu testenden Substanzen werden üblicherweise in einer Konzentration von 10⁻³M bis 10⁻⁶M dem Zellkulturmedium zugesetzt. Die zu testenden Substanzen bleiben in der Regel 2 bis 24 Stunden mit den transformierten Wirtszellen in Kontakt.

Anschließend werden die Zellen direkt in den Reportergen-Expressions-Assay eingesetzt oder Extrakte dieser Zellen nach ihrer Lyse, wie in Beispiel 4 angegeben ist. Die Expression des Reportergens in einer mit einer zu testenden Substanz behandelten Wirtszelle wird gemessen und verglichen mit einer Kontrolle, d.h. der Expression in Abwesenheit einer zu testenden Substanz. Durch Vergleich dieser beiden Meßwerte läßt sich sofort eine Aussage darüber machen, ob die zu testende Substanz die Reportergenexpression beeinflußt.

Das erfindungsgemäße Testverfahren hat den Vorteil, das es leicht und schnell durchzuführen und auszuwerten ist uns somit einen großen Probendurchsatz gestattet. Dadurch wird es möglich, ein effektives Massenscreening auf Substanzen mit Thrombinrezeptor antagonisierender Wirkung durchzuführen.

Die Erfindung wird durch die folgenden Beispiele weiter veranschaulicht.

### Beispiel 1

### Konstruktion des zusammengesetzten Promotors und Fusion mit dem Reportergen

a) Ausgehend von dem käuflichen Reporterkonstrukt pMAMneo-luc (Fa. Clontech, Kat.No.6171-1) wurde ein Enhancer/Promotorfreies Reporterplasmid konstruiert. Dazu wurde der die regulatorischen Sequenzen des RSV-LTRs und des MMTV-LTRs enthaltende Bereich in pMAMneo-luc durch Restriktionsspaltung mit NdeI und NheI deletiert und durch einen Polylinker, der die Erkennungssequenzen für die Restriktionsenzyme NdeI, MluI, NotI, SpeI und NheI enthält, ersetzt.
   Das so entstandene Reporterplasmid pneoLuc dient somit der Aufnahme regulatorischer Sequenzen in die Polylinkerregion. Der zusammengesetzte Promotor wurde aus vier synthetischen Oligonukleotiden hergestellt:
b) SEQ ID NO:1 entspricht der Promotorregion des Cytomegalovirus Immediate Early Gens von Position -67 bis +1 (Hennighausen L.G., Fleckenstein B.; EMBO J. 5, 1367-1371 (1986)). Zusätzlich enthält SEQ ID NO:1 zwei Erkennungssequenzen für Restriktionsendonukleasen: am 5'-Ende für NotI, am 3'-Ende für SpeI. SEQ ID NO:2 entspricht dem Gegenstrang zu SEQ ID NO:1.
c) SEQ ID NO:3 entspricht der vierfachen Wiederholung des cAMP response elements des vasoactive intestinal peptide (VIP) Gens (Tsukada, T. et al. J.Biol.Chem. 262, 8743-8747, 1987). Zusätzlich enthält SEQ ID NO:3 zwei Erkennungssequenzen für Restriktions-endonukleasen: am 5'-Ende für NdeI, am 3'-Ende für NotI. SEQ ID NO:4 entspricht dem Gegenstrang zu SEQ ID NO:3.
d) Zur Herstellung des rekombinanten Vektors wurden SEQ ID NO:1 und SEQ ID NO:2 hybridisiert und mit NotI/SpeI gespalten, sowie SEQ ID NO:3 und SEQ ID NO:4 hybridisiert und mit NdeI/NotI gespalten. Beide Produkte wurden dann in den mit NdeI und SpeI linearisierten Vektor pneoLuc aus Beispiel 1a) kloniert. Diese Klonierungsstrategie hat die richtige, d.h. funktionelle Orientierung der Einzelkomponenten zur Folge.

### Beispiel 2

Transformation und Selektion einer stabilen Zellinie.

CHO-K1 Zellen werden in DMEM/HamF12 (1:1)-Medium mit 10% FCS in einer Zelldichte von 10⁶ Zellen pro ml in 10 cm Petrischalen eingesät und über Nacht kultiviert. Die Transfektion erfolgt mit 2 µg des in Bsp.1d) aufgeführten Reporterkonstruktes in 10 µl Transfektam (Fa. Promega, Nr. E 1231), aufgenommen in 2 mL serumfreien Mediums über 6 Stunden.

Danach erfolgte ein Überführen in serumhaltiges Medium. Nach weiteren 42 Stunden werden die Zellen in Selektionsmedium (600 µg/ml G-418-Sulfat, Geneticin) überführt. Stabile Geneticin-resistente Zellklone wurden nach 10-14 Tagen isoliert.

### Beispiel 3

### Messung der Genaktivität

Zellen gemäß Beispiel 2 wurden in Mikrotiterplatten (Packard Viewplate No.600-5182) eingesät (1x10⁵ Zellen/well) und nach 16 h in DMEM/HamF12 (1:1)-Medium ohne FCS überführt. Nach weiteren vier Stunden werden Verdünnungen der zu testenden Substanzen zugegeben. Gleichzeitig wurde 0.01 - 0.1 NIHU/mL Thrombin zugegeben. Nur mit Thrombin behandelte Zellen dienten als Negativkontrolle. Der Test wurde nach 2-24 h durch die Lyse der Zellen beendet.

Lyse, Herstellung von Zellextrakten, sowie die Bestimmung der Luziferase-Aktivität erfolgten nach Angaben und mit Hilfe des Luziferase-Assay-Kits (Fa. Promega, Nr. E-1500). Im einzelnen wurden die Zellen durch Zugabe von 30 µl/well Lysispuffer (25 mM Tris-Phosphat, pH7,8; 2 mM DTT; 2 mM 1,2 Diaminocyclohexan-N,N,N',N'-tetraessigsäure, 10% Glycerol, 1% Triton X-100) aufgeschlossen (15 min. bei Raumtemperatur).

Zu diesem Zellextrakt wurden 50 µl Luciferase Assay Substrate (270 µM Coenzym A, 470 µM Luciferin, 530 µM ATP) zugegeben. Die Messung der Luziferase-Aktivität kann in herkömmlichen Luminometern durchgeführt werden; vorzugsweise erfolgt die Messung in einem 2-D-Luminometer der Fa. Hamamatsu.

### Beispiel 4

### Einsatz des Testsystems im "random-screening"

Das Assaysystem gemäß den Beispielen 1-3 wird eingesetzt für ein "random-screening" nach Substanzen, die spezifisch die Aktivierung des Thrombinrezeptors beeinflussen. Substanzen, die im Screening verwendet werden sollen, werden in Konzentrationen von 1-5 mM in DMSO gelöst und in geeigneten Verdünnungen gemäß Beispiel 3 in Tests eingesetzt.

## Patentansprüche

1. Testverfahren zur Identifizierung von Substanzen, die die Aktivierung des Thrombinrezeptors blockieren, welches folgende Schritte umfaßt:
a) Wahl einer Zellinie, die einen Thrombinrezeptor endogen exprimiert oder Herstellen einer Zellinie, die einen Thrombinrezeptor rekombinant stabil exprimiert,
b) Transfektion einer Wirtszelle nach Schritt a) mit einem rekombinanten Vektor, der
b1) einen Promotor enthält, der durch Erhöhung intrazellulärer Botenstoffe aktiviert wird, und
b2) ein Reportergen, funktionell mit dem in b1) beschriebenen Promotor verknüpft, trägt,
c) Etablieren von Zellen nach Schritt b), die
c1) den unter Schritt b) beschriebenen Vektor transient enthalten, oder
c2) den unter Schritt b) beschriebenem Vektor stabil integriert enthalten,
d) Exposition der Zellen aus Schritt c) mit der zu testenden Substanz,
e) Messung der Expression des Reportergens in den Zellen nach Schritt d).

2. Testverfahren nach Anspruch 1, dadurch gekennzeichnet, daß als Reportergen in Schritt b2) ein Luziferasegen verwendet wird.

3. Testverfahren nach Anspruch 1 oder 2, dadurch gekennzeichnet, daß als Zelle in Schritt a) die Zelle CHO-K1 verwendet wird.


SEQUENZPROTOKOLL
(1) ALLGEMEINE INFORMATION:
(i) ANMELDER:
(A) NAME: BASF Aktiengesellschaft
(B) STRASSE: Carl-Bosch-Strasse 38
(C) ORT: Ludwigshafen
(E) LAND: Bundesrepublik Deutschland
(F) POSTLEITZAHL: D-67056
(G) TELEPHON: 0621/6048526
(H) TELEFAX: 0621/6043123
(I) TELEX: 1762175170
(ii) ANMELDETITEL: Testverfahren zur Identifizierung von
Thrombinrezeptor-Antagonisten
(iii) ANZAHL DER SEQUENZEN: 4
(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)
(2) INFORMATION ZU SEQ ID NO: 1:
(i) SEQUENZ CHARAKTERISTIKA:
(A) LÄNGE: 75 Basenpaare
(B) ART: Nukleinsäure
(C) STRANGFORM: Einzel
(D) TOPOLOGIE: linear
(ii) ART DES MOLEKÜLS: DNS (genomisch)
(iii) HYPOTHETISCH: NEIN
(iv) ANTISENSE: NEIN
(vi) URSPRÜNLICHE HERKUNFT:
(A) ORGANISMUS: Human Cytomegalo Virus
(viii) POSITION IM GENOM:
(B) KARTENPOSITION: Immediate Early Gene Promotor
(xi) SEQUENZBESCHREIBUNG: SEQ ID NO: 1:
GGCCGCTGAC GCAAATGGGC GGTAGGCGTG TACGGTGGGA GGTCTATATA AGCAGAGCTC 60
GTTTAGTGAA CCGTA 75
(2) INFORMATION ZU SEQ ID NO: 2:
(i) SEQUENZ CHARAKTERISTIKA:
(A) LÄNGE: 75 Basenpaare
(B) ART: Nukleinsäure
(C) STRANGFORM: Einzel
(D) TOPOLOGIE: linear
(ii) ART DES MOLEKÜLS: DNS (genomisch)
(iii) HYPOTHETISCH: NEIN
(iii) ANTISENSE: JA
(vi) URSPRÜNLICHE HERKUNFT:
(A) ORGANISMUS: Human Cytomegalo Virus

(viii) POSITION IM GENOM:
(B) KARTENPOSITION: Immediate Early Gene Promotor
(xi) SEQUENZBESCHREIBUNG: SEQ ID NO: 2:
CTAGTACGGT TCACTAAACG AGCTCTGCTT ATATAGACCT CCCACCGTAC ACGCCTACCG 60
CCCATTTGCG TCAGC 75
(2) INFORMATION ZU SEQ ID NO: 3:
(i) SEQUENZ CHARAKTERISTIKA:
(A) LÄNGE: 106 Basenpaare
(B) ART: Nukleinsäure
(C) STRANGFORM: Einzel
(D) TOPOLOGIE: linear
(ii) ART DES MOLEKÜLS: DNS (genomisch)
(iii) HYPOTHETISCH: NEIN
(iv) ANTISENSE: NEIN
(vi) URSPRÜNLICHE HERKUNFT:
(A) ORGANISMUS: Homo sapiens
(xi) SEQUENZBESCHREIBUNG: SEQ ID NO: 3:
TATGCCCCAT GGCCGTCATA CTGTGACGTC CCCATGGCCG TCATACTGTG ACGTCCCCAT 60
GGCCGTCATA CTGTGACGTC CCCATGGCCG TCATACTGTG ACGTGC 106
(2) INFORMATION ZU SEQ ID NO: 4:
(i) SEQUENZ CHARAKTERISTIKA:
(A) LÄNGE: 108 Basenpaare
(B) ART: Nukleinsäure
(C) STRANGFORM: Einzel
(D) TOPOLOGIE: linear
(ii) ART DES MOLEKÜLS: DNS (genomisch)
(iii) HYPOTHETISCH: NEIN
(iv) ANTISENSE: JA
(vi) URSPRÜNLICHE HERKUNFT:
(A) ORGANISMUS: Homo sapiens
(xi) SEQUENZBESCHREIBUNG: SEQ ID NO: 4:
GGCCGCACGT CACAGTATGA CGGCCATGGG GACGTCACAG TATGACGGCC ATGGGGACGT 60
CACAGTATGA CGGCCATGGG GACGTCACAG TATGACGGCC ATGGGGCA 108